# EUROPÄISCHE PATENTANMELDUNG

(11) **EP 1 078 613 A2**
(43) Veröffentlichungstag der Anmeldung: **28.02.2001**
(21) Anmeldenummer: 00115892.2
(22) Anmeldetag: 25.07.2000
(51) Int. Cl.: A61F 5/453

(54) **Kondomurinal**

(30) Priorität: 26.08.1999 DE 19940639
(71) Anmelder: Spans, Wolfgang, 40489 Düsseldorf (DE)
(72) Erfinder: Spans, Wolfgang, 40489 Düsseldorf (DE)
(74) Vertreter: COHAUSZ HANNIG DAWIDOWICZ & PARTNER

(57) **Zusammenfassung**

Die Erfindung betrifft ein Kondomurinal zur lösbaren Befestigung am Glied einer männlichen Person, wobei ein erster Bereich zur Befestigung über die Eichel schiebbar ist und ein daran außen anschließender Bereich einen Trichter mit einem Anschluß für einen Urinbeutel aufweist Der erste Bereich ist zweilagig. Die erste innere Lage liegt zwischen der Eichel und der Innenseite der Vorhaut. Die zweite äußere Lage liegt an der Außenseite der Vorhaut an. Die zweite äußere Lage ist an ihrer Innenseite mit einer Klebstoffschicht versehen.

## Beschreibung

Die Erfindung betrifft ein Kondomurinal zur lösbaren Befestigung am Glied einer männlichen Person, wobei ein erster Bereich zur Befestigung über die Eichel schiebbar ist und ein daran außen anschließender Bereich einen Trichter mit einem Anschluß für einen Urinbeutel aufweist, wobei der erste Bereich zweilagig ist, und die erste innere Lage zwischen der Eichel und der Innenseite der Vorhaut liegt und die zweite äußere Lage an der Außenseite der Vorhaut anliegt.

Es ist bekannt, Kondomurinale ähnlich einem üblichen Kondom zu gestalten, so dass das Kondomurinal soweit über das Glied geschoben werden kann, dass es mittels einer selbsthaftenden inneren Klebefläche oder mit Klebestreifen am Penisschaft befestigbar ist. Ein solches Kondomurinal hat unter anderem folgende Nachteile:

Da der Glied innerhalb kurzer Zeit verschiedenen Schwellungszuständen unterliegt, kann es zu Einengungen bzw. dazu führen, dass das Kondom abrutscht. Dieses Problem besteht speziell bei älteren Männern mit stark retrahiertem Glied. Abgesehen vom mangelnden Tragkomfort entsteht ein Gefühl der Unsicherheit. Da das Glied längere Zeit komplett luftdicht abgeschlossen ist, weicht die Haut auf, was zu Hautläsionen führen kann. Das Entfernen des Kondoms wird als unangenehm empfunden, da immer Haare mit festgeklebt werden.

Aufgabe der Erfindung ist es, ein Kondomurinal der eingangs genannten Art so zu verbessern, dass es bei hohem Tragekomfort über längere Zeit einen sicheren Halt schafft.

Diese Aufgabe wird erfindungsgemäß dadurch gelöst, dass die zweite äußere Lage an ihrer Innenseite mit einer Klebstoffschicht versehen ist.

Ein solches Kondomurinal wird nur noch an der Vorhaut des männlichen Gliedes fixiert. Es bildet mit der Vorhaut ein geschlossenes System zur Urinableitung. Hierbei ist von Vorteil, dass die Vorhaut des männlichen Gliedes keiner aktiven Größenveränderung unterliegt. Passiv läßt die Vorhaut sich auf ihren natürlichen Umfang dehnen und durch einen formstabilen Ring in diesem Zustand fixieren. Die Befestigung des Kondoms durch Klebstoff an der Außenseite der Vorhaut garantiert einen sicheren Halt und absolute Dichtigkeit.

Die gesamte Klebefläche ist relativ gering, so dass Irritationen vermieden werden. Der Schwellungszustand des Gliedes ist für den Tragekomfort und die Dichtigkeit irrelevant. Der Zwischenraum zwischen Eichel und Vorhaut kann problemlos bei angelegtem Kondom mit milden Reinigungsmitteln gesäubert werden.

Durch entsprechende Konstruktion des Urinals sind Katheteruntersuchungen oder Selbstkatheterisierungen bei angelegtem Urinal möglich. Dieses System kann in vielen Fällen einen Dauerkatheter mit all seinen Risiken ersetzen.

Durch längere Tragezeiten werden Kosten gespart. Das System vermittelt dem Träger ein großes Gefühl der Sicherheit verbunden mit einem Optimum an Tragekomfort.

Vorteilhafte Ausgestaltungen der Erfindung sind in den Unteransprüchen aufgeführt.

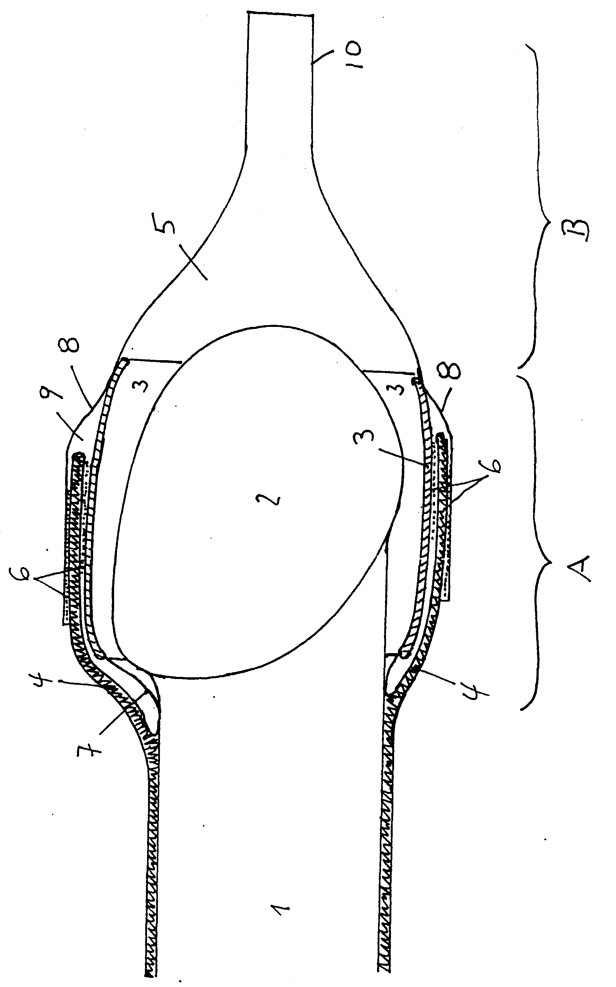

Ein Ausführungsbeispiel der Erfindung ist in der Zeichnung in einem Längsschnitt dargestellt und wird im folgenden näher beschrieben.

Um ein Kondomurinal an ein männliches Glied mit Penisschaft 1, Eichel 2 und Vorhaut 4 zu befestigen, weist dieses einen ersten Bereich A zur Befestigung und einen zweiten Bereich B zum Ableiten des Urins auf. Der erste Bereich A ist zweilagig gestaltet, das heißt er besitzt eine erste innere Lage 3, mit einem inneren formstabilen Ring. Diese innere Lage 3 kann auf der Eichel aufliegen und ist mit ihrer Außenseite an der Innenseite der Vorhaut 4 befestigt. Hierzu weist die Außenseite der inneren Lage 3 eine Klebstoffbeschichtung aus einem hautfreundlichen Klebstoff auf.

Über der vorzugsweise schlauchförmigen inneren Lage 3 liegt eine zweite äußere Lage 8 in Schlauchform, die an der inneren Lage an dem Ende der inneren Lage befestigt ist, die dem Penisschaft abgewandt ist. Damit bilden die zwei Lagen 3 und 8 einen zum Penisschaft hin offenen Zwischenraum 9, in dem die Vorhaut 4 einliegt.

Die zweite äußere Lage 8 ist an ihrer Innenseite mit einem hauffreundlichen Klebstoff 6 beschichtet, so dass sowohl die Innenhaut als auch die Außenhaut der Vorhaut 4 sicher fixiert sind.

Der zweite Bereich B zum Ableiten des Urins weist einen der Eichel nahen trichterförmigen Bereich (Rolltrichter) 5 und ein daran anschließenden Ansatz 10 zum Befestigen eines Schlauches bzw. eines Urinbeutels auf.

Der formstabile Ring 3 bietet immer noch Halt, wenn die Eichel herausgeglitten ist. Der Ring 3 kann aus zwei oder mehr Segmenten bestehen oder ein Spaltring sein.

## Patentansprüche

1. Kondomurinal zur lösbaren Befestigung am Glied (1, 2, 4) einer männlichen Person, wobei ein erster Bereich (A) zur Befestigung über die Eichel (2) schiebbar ist und ein daran außen anschließender Bereich (B) einen Trichter (5) mit einem Anschluß (10) für einen Urinbeutel aufweist, wobei der erste Bereich (A) zweilagig ist, und die erste innere Lage (3) zwischen der Eichel (2) und der Innenseite der Vorhaut (4) liegt und die zweite äußere Lage (8) an der Außenseite der Vorhaut (4) anliegt, **dadurch gekennzeichnet,** dass die zweite äußere Lage (8) an ihrer Innenseite mit einer Klebstoffschicht (6) versehen ist.

2. Kondomurinal nach Anspruch 1, **dadurch gekennzeichnet,** dass die erste innere Lage (3) an ihrer Außenseite mit einer Klebstoffschicht (6) versehen ist.

3. Kondomurinal nach einem der Ansprüche 1 oder 2, **dadurch gekennzeichnet,** dass die erste innere Lage (3) einen formstabilen Ring bildet oder aufweist.
